# EUROPEAN PATENT APPLICATION

(11) **EP 1 867 607 A1**
(43) Date of publication of application: **19.12.2007**
(21) Application number: 06745369.6
(22) Date of filing: 16.03.2006
(51) Int. Cl.: C02F 1/68, A61K 35/02

(54) **PROCESS AND KIT FOR FORMATION OF ACTIVE HYDROGEN WATER, GYPSUM FEEDER FOR THE FORMATION, ACTIVE HYDROGEN FORMING MATERIALS AND PROCESS FOR THE PRODUCTION OF THE MATERIALS**

(30) Priority: 17.03.2005 JP 2005077851
(71) Applicant: Shiga, Seiki, Yokohama-shi, Kanagawa 226-0028 (JP)
(72) Inventor: Shiga, Seiki, Yokohama-shi, Kanagawa 226-0028 (JP)
(74) Representative: HOFFMANN EITLE
(86) International application number: PCT/JP2006/305249
(87) International publication number: WO 2006/098405

(57) **Abstract**

An active hydrogen-dissolved water generator 10 contains a container 11 such as a PET bottle, a first case 12 filled with magnesium metal, and a second case 13 filled with gypsum. The first case 12 and the second case 13, and a drinking water 15 are introduced into the container 11 and stored therein to generate an active hydrogen-dissolved water. By adding calcium sulfate or magnesium sulfate, and a magnesium metal particle to the drinking water 15, an active hydrogen-rich water can be generated and the retention of the active hydrogen generation function can be improved.

## Description

### Technical Field

The present invention relates to an active hydrogen water (i.e., water containing active hydrogen dissolved therein), and particularly to a method for generating an active hydrogen-dissolved water effective for eliminating active oxygen in human/animal bodies, an active hydrogen-dissolved water generator, and a gypsum supply member therefor, and an active hydrogen generating material and a production method thereof.

### Background Art

Recently, it has been considered that active oxygen causes various diseases in human and animal bodies due to its strong oxidizing properties. For example, the active oxygen is suspected to be the chief cause of allergic disease, diabetes mellitus, hypertension, cancer, invasive viral disease, etc. Thus, a theory that an active hydrogen-dissolved water, which contains a larger amount of active hydrogen (atomic hydrogen) as compared with natural water or tap water (drinking water), is effective for eliminating the active oxygen has been published and attracted much attention in the medical world.

The artificial water containing a larger amount of active hydrogen has been known as electrolytic reduced water or alkaline electrolyzed water, and can be obtained by utilizing electrolysis of a drinking water (see, e.g. Patent Document 1).
However, apparatus used in the method of utilizing electrolysis has an electrolytic bath, an electric power supply, various distributing water pipes, etc., thereby resulting in a complicated structure. Thus, this method cannot provide the active hydrogen-dissolved water simply and inexpensively, and an extremely simpler method for generating the active hydrogen-dissolved water is proposed. In this method, a drinking water and a magnesium metal (particle) are reacted to generate a hydrogen gas, whereby the drinking water is converted to a hydrogen rich water containing a large amount of hydrogen (see, e.g. Patent Document 2).
Patent Document 1: JP-A-9-77672
Patent Document 2: JP-A-2004-041949

### Disclosure of the Invention

### Problems that the Invention is to Solve

Though the generating apparatus for the above electrolysis method is commercially available as a medical device, it is extremely expensive for personal purchase.
Further, the active hydrogen-dissolved water generated by the above apparatus has a remarkably short storage life. The effects of this active hydrogen-dissolved water disappear generally in approximately 2 to 3 hours, which is a time taken for the remaining active hydrogen content to reach a value 0. 005 ppm. This is because the active hydrogen content of the active hydrogen-dissolved water generated by the electrolysis declines exponentially with the storage period. Therefore, it is also difficult to generate a large amount of the active hydrogen-dissolved water at one time by the above specific generating apparatus, and store and use the water inexpensively.
For the above reasons, it is difficult to widely popularize the active hydrogen-dissolved water for human and animal medicines, etc. at present.

On the other hand, the method disclosed in Patent Document 2 utilizes a chemical reaction between the magnesium metal and the drinking water, represented by the following formula.

[Chemical Formula 1] Mg + 2H₂O → Mg(OH)₂ + 2H → Mg(OH)₂ + H₂

However, in the above chemical reaction, magnesium hydroxide (Mg(OH)₂) hardly soluble in water is formed on the magnesium metal particle. And the magnesium hydroxide layer inhibits the chemical reaction between the magnesium metal particle surface and water. As a result, the generation of the active hydrogen is extremely reduced over time. Thus, it is necessary that, after a certain period of use, the magnesium metal particle is soaked in a vinegar, whereby the magnesium hydroxide layer formed on the particle surface is dissolved and removed regularly.
Thus, the method of Patent Document 2 is disadvantageous in the retention of the active hydrogen generating function, so that the magnesium hydroxide layer, which is a factor for the disadvantage, has to be regularly removed. Further, also the magnesium metal particle surface is dissolved and etching-removed by the vinegar in the process of removing the magnesium hydroxide layer, and thus the magnesium metal particle is disadvantageously wasted. Furthermore, the method is disadvantageous also in that it is difficult to increase the active hydrogen content of the active hydrogen-dissolved water, thereby improving its effects.

Under the circumstances, an object of the present invention is to achieve such a long-lived active hydrogen-dissolved water that the active hydrogen content is increased, the effects of the active hydrogen is maintained for a longer time, and the retention of the active hydrogen generation is improved.
Further, another object of the invention is to provide a highly effective active hydrogen-dissolved water using a simple method.

### Means for Solving the Problems

The inventor has studied in detail on the effects of hydrated ions of alkaline earth metals such as calcium and magnesium, in-water mineral components, on active hydrogen generation in water. As a result, the inventor has found that the above hydrated ions can efficiently increase active hydrogen content in water for the first time. Further, the inventor has found that the formation of a magnesium hydroxide layer on the magnesium metal surface can be greatly reduced by adding a sulfate ion derived from calcium sulfate (gypsum), magnesium sulfate, etc. to water. The present invention has been accomplished based on these findings.

In view of achieving the above objects, the method of the invention for generating an active hydrogen-dissolved water may contain the step of adding magnesium metal, and gypsum or magnesium sulfate to water such as a so-called drinking water, thereby generating a water containing an active hydrogen.

Alternatively, the method of the invention for generating an active hydrogen-dissolved water may contain the step of adding a mixture of magnesium metal and gypsum to water, thereby generating a water containing an active hydrogen.
In the invention, the gypsum is hemihydrate gypsum, dihydrate gypsum, or anhydrous gypsum.

By using the above constituents, the active hydrogen in the active hydrogen-dissolved water is bonded to a hydrated calcium ion or hydrated magnesium ion dissociated from the dissolved calcium sulfate or magnesium sulfate, whereby the active hydrogen content is increased, and thus the effects of the active hydrogen-dissolved water is enhanced.

Though magnesium hydroxide is formed on a surface of the magnesium metal soaked in water, the magnesium hydroxide is converted to a readily soluble magnesium ion by a sulfate ion dissociated from the dissolved calcium sulfate or magnesium sulfate, and dissolved and removed. Thus, the active hydrogen is constantly generated by the reaction between the magnesium metal and water, and the regular process of soaking the magnesium metal in a vinegar, thereby dissolving and removing the magnesium hydroxide, is not required in the invention though the process is necessary in conventional methods.

The active hydrogen-dissolved water generator of the invention may contain a water container, a member filled with magnesium metal, and a member filled with gypsum, the members being placed in the container.

Alternatively, the active hydrogen-dissolved water generator of the invention may contain a water container and a member filled with magnesium metal and gypsum, the member being placed in the container.

Alternatively, the active hydrogen-dissolved water generator of the invention may contain a water container and a member filled with a mixture of magnesium metal and gypsum, the member being placed in the container.

Alternatively, the active hydrogen-dissolved water generator of the invention may contain a water container, a member filled with magnesium metal, and a member filled with magnesium sulfate, the members being placed in the container.

In a preferred embodiment of the invention, a light-shielding layer is formed on a surface of the member to prevent the gypsum from being exposed to light.

By using the above constituents, the active hydrogen-dissolved water can be remarkably easily generated, and thus an inexpensive active hydrogen-dissolved water product can be widely provided.

The gypsum supply member of the invention for supplying the calcium sulfate contains a case into which water is introduced, and gypsum or a mixture of magnesium metal and gypsum put in the case. A light shielding layer may be formed on the case to prevent the gypsum put in the case from being exposed to light. Alternatively, the gypsum can be prevented from being exposed to light by using a light shielding material for the case instead of forming the light shielding layer.

The active hydrogen generating material of the invention contains a solid matter prepared by mixing a magnesium metal powder with gypsum, and the material is added to water to generate active hydrogen. The magnesium metal powder has a particle diameter of 0.05 to 0.15 mm, and the gypsum is hemihydrate gypsum, dihydrate gypsum, or anhydrous gypsum.

The method of the invention for producing the active hydrogen generating material contains the steps of mixing gypsum, a magnesium metal powder, and water at a first temperature to form a thick mixture, and solidifying the thick mixture at a second temperature higher than the first temperature.

In a preferred embodiment of the invention, the gypsum is in a powdery form, and the first temperature is within the range of 10°C to 18°C, and the second temperature is room temperature.

In a preferred embodiment of the invention, the water has a pH value of 10 to 13, and sodium hydroxide, calcium hydroxide, or potassium hydroxide is dissolved in the water. It is preferred that alternatively the water is boiled once before the mixing step.

By using the above constituents, the active hydrogen generating material is formed as a solid mixture, in which the magnesium metal powder is approximately uniformly dispersed in the gypsum. Further, the mixture has a dense structure, and when the mixture is put in water to generate the active hydrogen-dissolved water, the gypsum and the magnesium metal powder are eluted from the periphery of the mixture. Thus, the retention of the active hydrogen generation is increased, and a high concentration of the active hydrogen is stably generated.

### Advantage of the Invention

According to the present invention, the dissolved active hydrogen content is increased, and the effect retention time of the active hydrogen-dissolved water is made longer. Further, the formation of the magnesium hydroxide layer is prevented on the magnesium metal particle surface, and the retention of the active hydrogen generation is improved. Furthermore, the active hydrogen-dissolved water can be generated by a simple method, so that an inexpensive active hydrogen-dissolved water can be widely provided.

### Brief Description of the Drawings

Fig. 1 is a schematic structural view showing an active hydrogen-dissolved water generator according to Embodiment 1 of the present invention.
Fig. 2 is a view showing an active hydrogen generating member according to Embodiment 1 of the invention.
Fig. 3 is a side view showing a gypsum supply member according to Embodiment 1 of the invention.
Fig. 4 is a transverse sectional view showing the gypsum supply member according to Embodiment 1 of the invention.
Fig. 5 is a graph showing an example of temporal change in the dissolved hydrogen content of an active hydrogen-dissolved water to illustrate the advantageous effects of the invention.
Fig. 6 is a graph showing an effect of calcium sulfate or magnesium sulfate addition in the invention.
Fig. 7 is a graph showing an example of temporal change in the active hydrogen content of an active hydrogen-dissolved water to illustrate the advantageous effects of the invention.
Fig. 8 is a graph showing a temporal change in the dissolved hydrogen content of an active hydrogen-dissolved water to illustrate the advantageous effects of Embodiment 1 of the invention.
Fig. 9 is a schematic structural view showing an active hydrogen-dissolved water generator according to Embodiment 2 of the invention.
Fig. 10 is a view showing a hydrogen generating solid matter according to Embodiment 2 of the invention.
Fig. 11 is a flow chart of a method for producing the hydrogen generating solid matter according to Embodiment 2 of the invention.
Fig. 12 is a side view showing a hydrogen generating solid matter supply member according to Embodiment 2 of the invention.
Fig. 13 is a transverse sectional view showing the hydrogen generating solid matter supply member according to Embodiment 2 of the invention.
Fig. 14 is a graph showing a temporal change in the dissolved hydrogen content of an active hydrogen-dissolved water to illustrate the advantageous effects of Embodiment 2 of the invention.
Fig. 15 is a view showing a gypsum supply member according to Embodiment 3 of the invention.
Fig. 16 is a side view showing a hydrogen generating solid matter or gypsum supply member according to a modification embodiment of the invention.
Fig. 17 is a transverse sectional view showing the hydrogen generating solid matter or gypsum supply member according to the modification embodiment of the invention.

### Best Mode for Carrying Out the Invention

Some preferred embodiments of the present invention will be described below with reference to the drawings. It should be noted that, in the drawings, the same or similar parts are represented by the same numerals, and duplicate explanations therefor are omitted.

### (Embodiment 1)

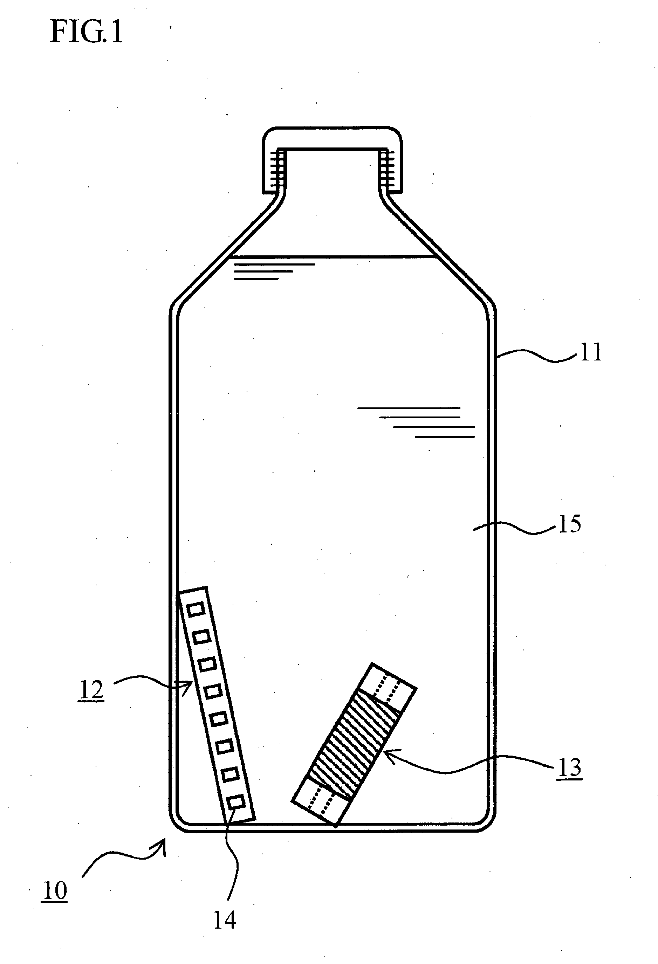

Fig. 1 is a schematic structural view showing an active hydrogen-dissolved water generator, which is an example for illustrating the method of the invention for generating an active hydrogen-dissolved water.

As shown in Fig. 1, the active hydrogen-dissolved water generator 10 has a container 11 such as a PET bottle, a first case 12 filled with magnesium metal, and a second case 13 filled with gypsum. A drinking water 15 is put in the container 11 and stored together with the first case 12 and the second case 13.

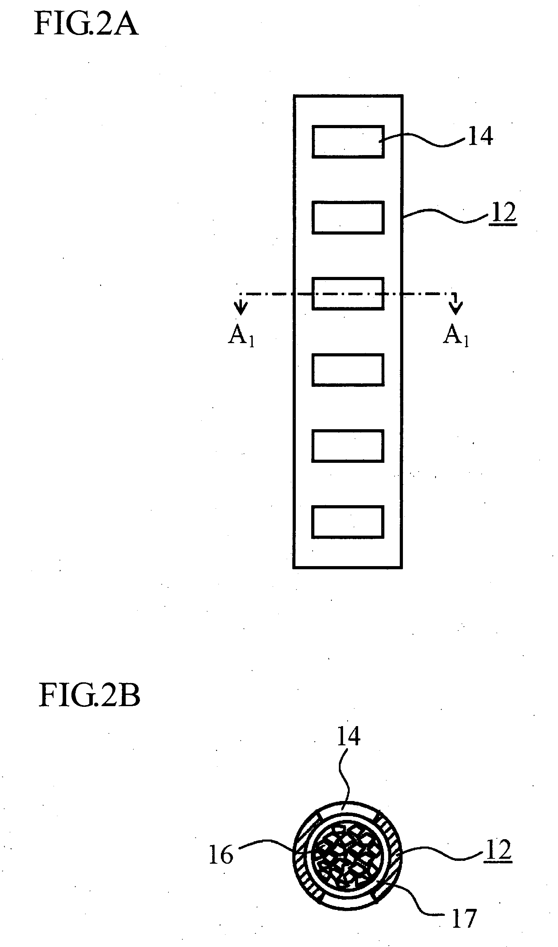

In a preferred embodiment, as shown in Figs. 2A and 2B, the first case 12 has a stick shape and is used as an active hydrogen generating member. Fig. 2A is a side view of the first case 12, and Fig. 2B is a sectional view along the A₁-A₁ arrow line in Fig. 2A.
As shown in Fig. 2, the first case 12 is composed of a thin cylindrical plastic material having a transverse section of a circle, and openings 14 through which a liquid is moved in and out the case are formed on the side wall. The interior portion of the first case 12 is communicated with the outside drinking water 15 by the openings 14. A bag 17 composed of a water permeable material such as a nonwoven fabric is filled with magnesium particles 16, and is placed in the first case 12.

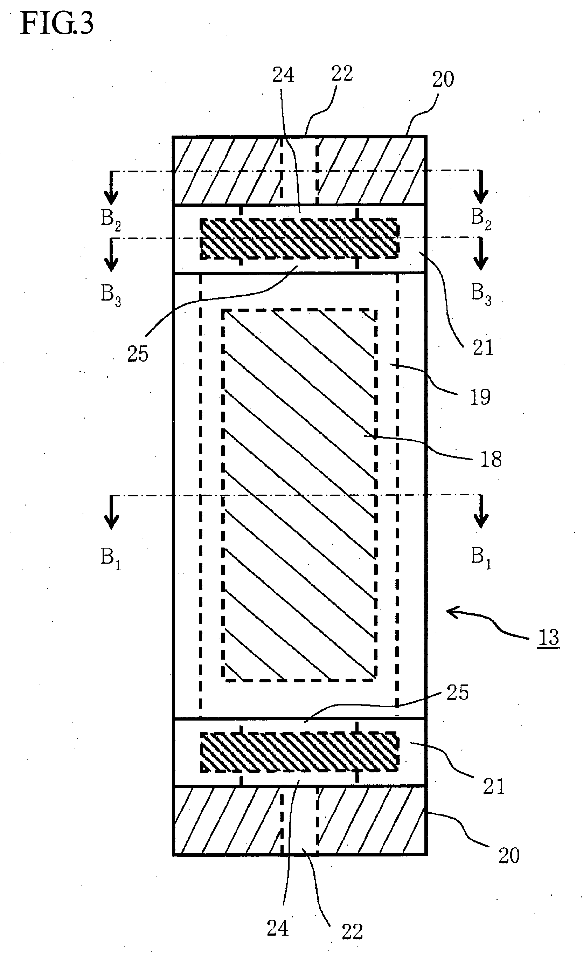

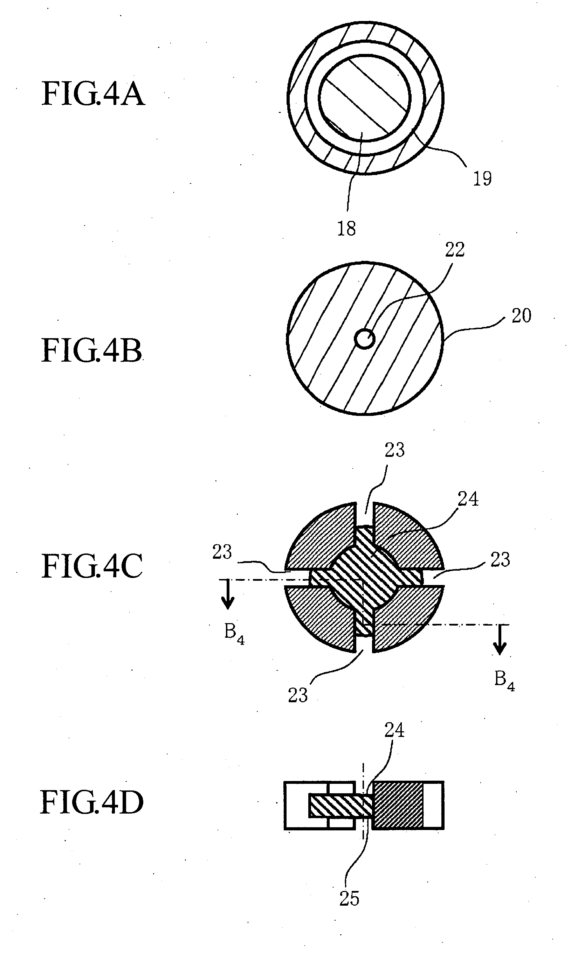

The second case 13 used as a gypsum supply member has a structure shown in Figs. 3 and 4. Fig. 3 is a side view of the second case 13, Fig. 4A is a transverse sectional view along the B₁-B₁ arrow line in Fig. 3, Fig. 4B is a transverse sectional view along the B₂-B₂ arrow line in Fig. 3, and Fig. 4C is a transverse sectional view along the B₃-B₃ arrow line in Fig. 3. Fig. 4D is a longitudinal sectional view along the B₄-B₄ arrow line in Fig. 4C.
As shown in Fig. 3, for example, the second case 13 is composed of a cylindrical vinyl chloride material, and has a packing chamber 19 in which a gypsum 18 is placed, and has a couple of first water flow tops 20 and second water flow tops 21 which the packing chamber 19 is vertically sandwiched between.
As shown in Fig. 4A, the gypsum 18 represented by oblique lines is placed inside the packing chamber 19. Further, as shown in Fig. 4B, a hole 22 bored into a cylindrical shape is formed in each first water flow top 20, and as shown in Fig. 4C, depressions 24 and 25 are formed on the top and bottom of each second zigzag water flow top 21, and flow channels 23 are formed at 4 positions of the top periphery. In this structure, the drinking water 15 passes through the hole 22, the depression 24, the flow channels 23, and the depression 25, to reach the gypsum packing chamber 19 shown in Fig. 3.

Then, the method and mechanism of generating the active hydrogen-dissolved water by using the above generator will be described below. When an ambient-temperature or cooled drinking water 15 is added to the container 11 with the first case 12 and the second case 13 as shown in Fig. 1, the drinking water 15 is reacted with the magnesium particle 16 in the first case 12, and the active hydrogen is generated in the first case 12, according to following Chemical Formula 1.

[Chemical Formula 1] Mg + 2H₂O → Mg(OH)₂ + 2H → Mg(OH)₂ + H₂

At the same time, the surface of the gypsum placed in the packing chamber 19 is dissolved in the drinking water 15 introduced from the hole 22. Then, the gypsum of calcium sulfate (CaSO₄) is dissociated into a sulfate ion (SO₄²⁻) and a hydrated calcium ion (Ca(H₂O)_{6.2})²⁺ with coordinate 6.2 water molecules on average, and large amounts of the ions are accumulated in the packing chamber 19. The hydrated ion is partly dispersed through the hole 22 into the container 11, and is supplied in an amount appropriate for the drinking water 15.

The concentration of the active hydrogen generated in the first case 12 is increased by the dissolved and dissociated hydrated calcium ion in the drinking water 15 in this manner. Thus, a high-concentration, active hydrogen-dissolved water is generated.

Next, the effect of increasing the active hydrogen content of the drinking water 15 due to the hydrated ion will be described in detail below. The inventor weighed calcium sulfate and magnesium sulfate (MgSO₄), and dissolved and electrolyzed them in a tap water, and thus carried out a trial experiment on the temporal change of the active hydrogen content in the cathode water.
In the experiment, the electrolysis was repeated under various amounts of the calcium sulfate and magnesium sulfate added to the tap water, and the temporal change of the dissolved hydrogen (H₂, H*) content in the cathode water was examined after the electrolysis. By the above addition, the calcium sulfate is dissolved in the cathode water in the form of the hydrated calcium ion and the sulfate ion, and in the same manner, the magnesium sulfate is dissolved in the form of the hydrated magnesium ion (Mg(H₂O)₆)²⁺ with coordination 6 water molecules and the sulfate ion.
The dissolved hydrogen was measured by a dissolved hydrogen meter KM2100DH manufactured by Kyoei Denshi Kenkyusho. The dissolved hydrogen meter measures the total amount of the hydrogen molecule (H₂) or the active hydrogen (H*).

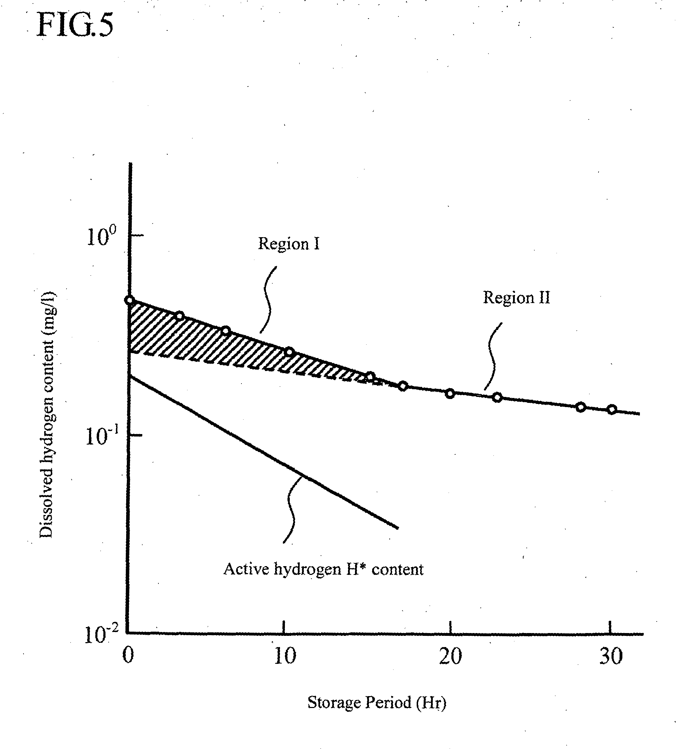

Fig. 5 is a graph showing such an example that a hemihydrate gypsum was added to a tap water, calcium sulfate was dissolved in the tap water at a concentration of 2.1 × 10⁻³ mol/l (water hardness 210), i. e. 210 mg/l based on the calcium carbonate, and electrolysis was carried out. The dissolved hydrogen content is plotted along the ordinate axis, and the storage period of the cathode water is plotted along the abscissa axis. As shown by white circles in Fig. 5, the dissolved hydrogen content of the cathode water is exponentially reduced with 2 different time constants. Thus, the active hydrogen (H*) is reduced in the period of Region I represented by the oblique lines in the drawing, and the hydrogen molecule (H₂) is moderately reduced with a larger time constant than that of the active hydrogen (H*) in the period of Region II in the drawing.

In Fig. 5, the region of the oblique lines is the difference between the dissolved hydrogen content and the hydrogen molecule content, obtained by expanding the line of Region II to Region I, and considered to substantially represent the temporal decay of the active hydrogen. Thus, the difference is considered as the change in the active hydrogen H* content and represented in the graph. As shown in the graph, the active hydrogen H* decay time constant of Region I represents that the active hydrogen is rapidly reduced in 10 to 11 hours. The change of the H* content was confirmed also by a method of soaking tungsten oxide in the cathode water and measuring the color change time of the tungsten oxide surface. The change of the H* content obtained by this method was qualitatively equal to the change obtained from the above difference.
Thus, graphs similar to Fig. 5 were obtained by changing the amount of the hemihydrate gypsum or magnesium sulfate dissolved in the tap water, and the change in the H* content was obtained from the above difference of each graph.

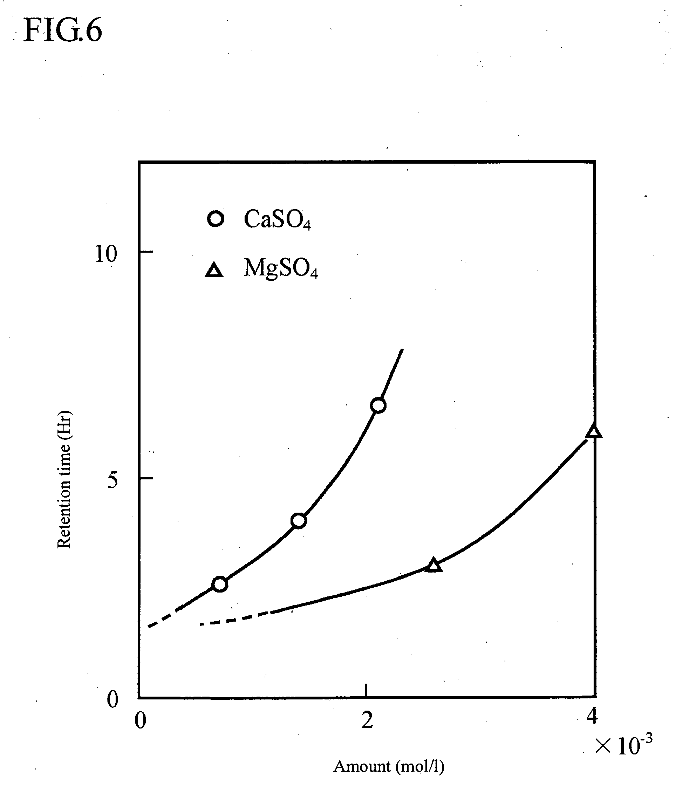

Fig. 6 is a graph showing an effect of the hydrated ion addition, obtained by changing the amount. In Fig. 6, the retention time of the H* content obtained by the above difference is plotted along the ordinate axis, and the amount is plotted along the abscissa axis. It should be noted that the retention time of the H* content is the time for reducing the dissolved H* content to 100 ppb by mass.
It is clear from Fig. 6 that both the calcium sulfate and magnesium sulfate have the effect of increasing the retention time of the active hydrogen, and the effect is improved with the increase in the amount of the sulfate. Further, also it is clear that the calcium sulfate has the retention time increasing effect greater than that of the magnesium sulfate. For example, the active hydrogen retention time is approximately 1 Hr when the calcium sulfate is not added at all, and in contrast, the retention time is increased to at least six times the value, approximately 6.5 Hr, when 2.1 × 10⁻³ mol/l of the calcium sulfate is dissolved. Further, when 4 × 10⁻³ mol/l of the magnesium sulfate is dissolved, the retention time is increased to about 6 Hr.

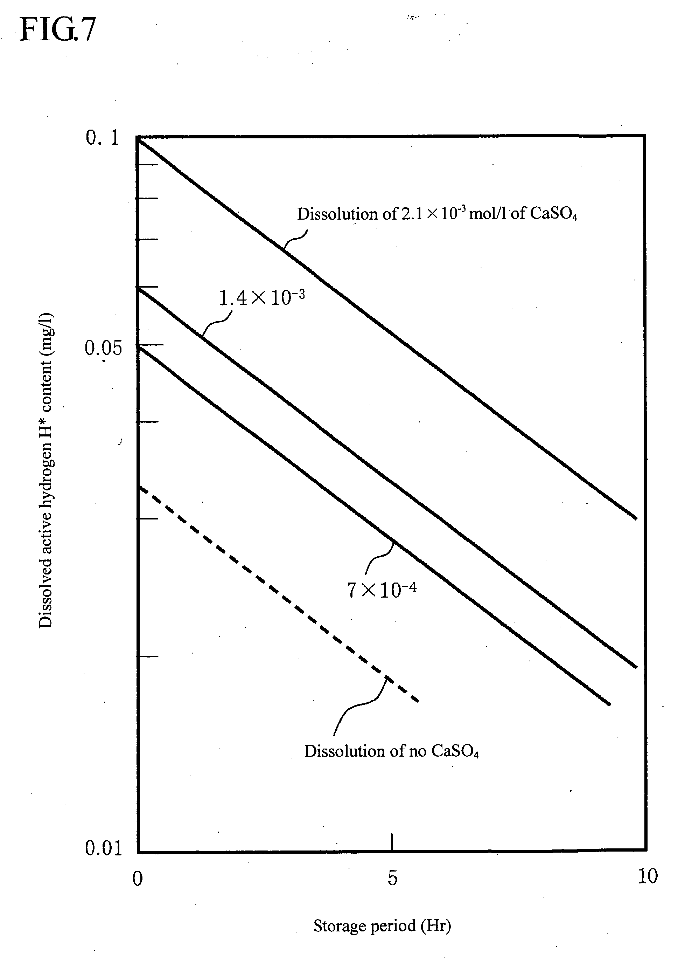

Further, the inventor has weighed a hemihydrate gypsum, dissolved the hemihydrate gypsum and a magnesium metal piece in a tap water, removed the magnesium metal piece from the tap water after 48 hours, and examined the temporal change in the dissolved active hydrogen H* content in the same manner as the description of Fig. 5. The results are shown in Fig. 7.
In Fig. 7, the tap water storage period (hour) after removing the magnesium metal piece is plotted along the abscissa axis, and the H* content is plotted along the ordinate axis. The unit quantity 1 mg/l on the ordinate axis corresponds to 1 ppm by mass. The amount of the dissolved calcium sulfate is used as a parameter.
It is clear from Fig. 7 that the H* content of the tap water is exponentially reduced, similarly to the case of the above described cathode water. Further, it is clear that the H* content of the tap water is increased as the amount of the dissolved magnesium sulfate (the parameter) is increased to 7 × 10⁻⁴, 1.4 × 10⁻³, and 2.1 × 10⁻³ mol/l.

In Embodiment 1, the first case 12 filled with the magnesium metal particles and the second case 13 filled with the gypsum are soaked in the drinking water 15, whereby the calcium sulfate added to the drinking water 15 is dissolved therein and the active hydrogen is generated according to Chemical Formula 1. Therefore, an active hydrogen-dissolved water having a high active hydrogen content described for Figs. 5 to 7 can be easily generated.
The mechanism of increasing the active hydrogen content due to the hydrated ion of the alkaline earth metal is still unclear. The inventor believes that, though the active hydrogen is converted to hydrogen molecule (H₂) and disappears in a very short period of time in water under a normal condition, the active hydrogen is bonded to the hydrated ion to form a hydrated ion adduct, etc. having a relatively long life in the invention. Further, it is believed that the amount of the hydrated ion adduct is increased with the amount of the calcium sulfate added to the drinking water, resulting in the above effect.

In Embodiment 1, the retention of the active hydrogen generation according to Chemical Formula 1 is increased in the drinking water 15. Thus, though the active hydrogen bonded to the hydrated ion is reduced over time as described on Fig. 7, the active hydrogen is further generated and a certain amount of the active hydrogen continuously exists in the drinking water 15.

In Embodiment 1, the gypsum is dissolved and dissociated to generate the sulfate ion, and the sulfate ion acts to dissolve the magnesium hydroxide formed on the magnesium particles 16 according to following Chemical Formula 2.

[Chemical Formula 2] Mg(OH)₂ + Ca²⁺ + SO₄²⁻ → Mg²⁺ + SO₄²⁻ + Ca²⁺ + 2OH⁻

It is confirmed that, when the magnesium metal and the gypsum are added to the drinking water, the calcium sulfate acts to dissolve the magnesium hydroxide and the hydrated magnesium (Mg) ion content of the drinking water is increased as shown in Table 1. Shown in Table 1 are values measured by a magnesium hydroxide precipitation method 1 day after the addition. In the case of adding no calcium sulfate, the hydrated Mg ion content is 4 × 10⁻⁵ mol/l. In contrast, in the case of adding 5 × 10⁻⁴ mol/l of the calcium sulfate, the hydrated Mg ion content is greatly increased to 2 × 10⁻⁴ mol/l, and it is clear that the magnesium hydroxide is easily dissolved.

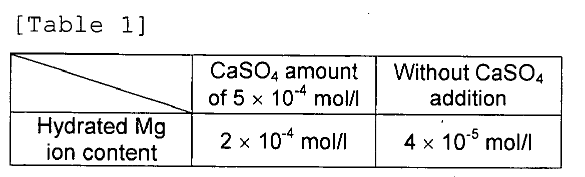

As described above, in Embodiment 1, the magnesium hydroxide is hardly formed on the magnesium particles 16 contained in the first case 12, and the disadvantageous decrease over time of Chemical Formula 1 reaction due to the magnesium hydroxide is drastically reduced.

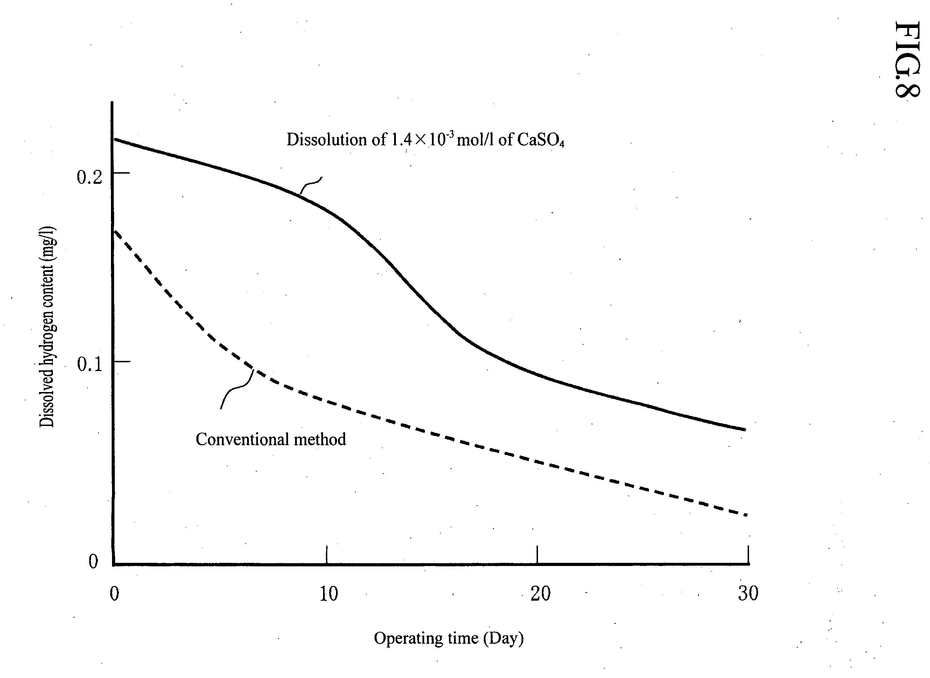

The above effect will be described with reference to Fig. 8. The method of generating an active hydrogen-dissolved water by dissolving a hemihydrate gypsum in a tap water as described in Embodiment 1 is compared with a conventional method of generating an active hydrogen-dissolved water by soaking a magnesium metal piece in a tap water according to Patent Document 2 in Fig. 8. Fig. 8 is a graph showing temporal changes in the dissolved hydrogen contents (total contents of hydrogen molecule and active hydrogen) of the active hydrogen-dissolved waters. The dissolved hydrogen contents were measured by a dissolved hydrogen meter KM2100DH. The operating time (day) of each active hydrogen-dissolved water is plotted along the abscissa axis, and the dissolved hydrogen content is plotted along the ordinate axis.

In Fig. 8, the solid line represents an example of the results of dissolving 1.4 × 10⁻³ mol/l (hardness 140) of the hemihydrate gypsum and soaking the magnesium metal particle therein. The dotted line represents an example of the results of the conventional method of soaking only the magnesium metal particle in the tap water.
In the conventional method, the dissolved hydrogen content is remarkably reduced in 10 days of the operating time. It is clear from the result that a magnesium hydroxide layer grows notably to reduce the hydrogen generation according to Chemical Formula 1 in the period. However, the dissolved hydrogen content is moderately reduced at a substantially constant rate after the 10 days.
As compared with the conventional method, in the method of dissolving the gypsum, the dissolved hydrogen content is higher, and particularly, the content is reduced at a lower rate in the operating time of 10 days. Further, after 20 days operating, the rate of the dissolved hydrogen content reduction becomes substantially equal to that of the conventional method. It should be noted that, even after 30 days operation, the dissolved hydrogen content in this case is equal to that in the conventional method after 10 days.

It is clear that, in Embodiment 1, the retention of the active hydrogen generation is greatly improved. Further, the process of soaking the magnesium particle 16 in a vinegar after the first case 12 is used for a predetermined time (e.g. at the rate of 2 times/week), thereby dissolving and removing the magnesium hydroxide, is not required in the invention, though the process is necessary in the conventional method.

In Embodiment 1, it is preferred that the amount of the gypsum dissolved is controlled. This is because the drinking water is converted to a so-called hard water by dissolving the gypsum, and a larger amount of the dissolved gypsum results in flavor deterioration. In general, the gypsum can be remarkably easily dissolved in water. For example, in the case of a dihydrate gypsum, about 7 × 10⁻³ mol/l (hardness 700) of calcium sulfate is dissolved in a drinking water in one day under a room illumination at room temperature (approximately 20°C), the saturated amount of the calcium sulfate dissolved is near 1.4 × 10⁻² mol/l at room temperature.

In the generation of the active hydrogen-dissolved water described using Fig. 1, by using the second case 13 shown in Figs. 3 and 4, the amount of the calcium sulfate eluted into the drinking water 15 can be easily controlled. In Fig. 3, the eluted calcium sulfate of the gypsum is dissociated into hydrated Ca²⁺ and SO₄²⁻ in the packing chamber 19. Because the zigzag water flow top 21 is communicated with outside through the hole 22, the dissociated ions are accumulated in the packing chamber 19, the concentrations of the ions are increased locally inside the zigzag water flow top 21, and thus the elution of the gypsum can be reduced.
Further, the elution can be reduced by forming a shielding layer, thereby preventing the packing chamber 19 of the second case 13 shown in Fig. 3 from being exposed to light.

The amount of the gypsum dissolved can be controlled at 2 × 10⁻³ mol/l (hardness 200) or less 1-week after the second case 13 is filled with an anhydrous gypsum or dihydrate gypsum, etc. and soaked in the drinking water in this manner. Incidentally, in the drinking water quality standard of Japan, the water hardness of 300 or less is satisfactory.

Then, a preferred embodiment of using the second case 13, which is a member for supplying the gypsum to the drinking water, and generating the active hydrogen-dissolved water will be described below.

A PET bottle having a volume of 1.5 to 2 L is used as the container 11, the first case 12 filled with the magnesium particles 16 and the second case 13 filled with the gypsum 18 such as a gypsum or a dihydrate gypsum are put in the PET bottle, and the PET bottle is filled with a tap water. It is preferred that chlorine in the tap water is removed by a commercially-available simple water purifier, etc. By storing the PET bottle in a room or a refrigerator for about half a day while protecting from direct sunlight, a sufficiently good-tasting active hydrogen-dissolved water can be generated. Then, this active hydrogen-dissolved water is used for drinking. When the active hydrogen-dissolved water in the PET bottle is used up, a tap water may be added to the PET bottle to continue the generation of the active hydrogen-dissolved water. Incidentally, it is preferred that, 3 days after putting the first case 12, the second case 13, and the tap water in the PET bottle, all the remaining active hydrogen-dissolved water is discarded and another tap water is added. This is because, 3 or more days after the generation, the content of the dissolved calcium exceeds 1 × 10⁻³ mol/l (hardness 100), thereby resulting in flavor deterioration.

The second case 13 filled with the gypsum can be continuously used in this manner generally over 2 to 2.5 months. On the other hand, the first case 12 filled with the magnesium particles can be continuously used over 1 month. The process of regularly washing the first case 12 with a vinegar is not required at all in this operating period. When all the gypsum 18 in the second case 13 is consumed, the member may be discarded or reused by replenishing the lacked gypsum, etc.

### (Embodiment 2)

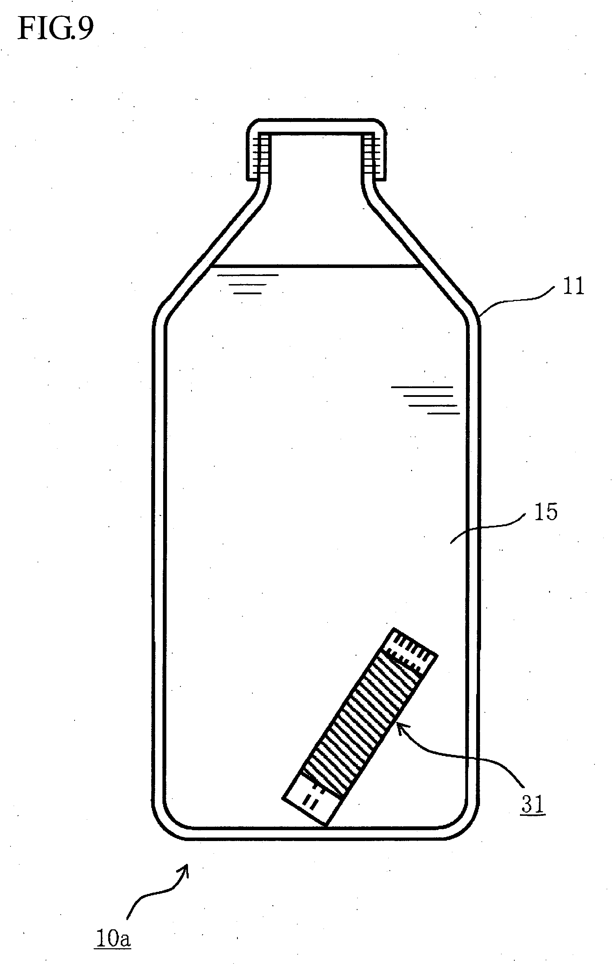

Fig. 9 is a schematic structural view showing another active hydrogen-dissolved water generator, an example for illustrating the method of the invention for generating an active hydrogen-dissolved water. Embodiment 2 is characterized by using a solid mixture of a gypsum and magnesium metal particles.

As shown in Fig. 9, the active hydrogen-dissolved water generator 10a has a container 11 such as a PET bottle, and a common case 31 filled with a hydrogen generating solid matter composed of the mixture of the gypsum and the magnesium particles to be hereinafter described. The common case 31 and a drinking water 15 are added to and stored in the container 11.

The method and mechanism of generating an active hydrogen-dissolved water using the generator 10a are the same as those of Embodiment 1. Thus, when an ambient-temperature or cooled drinking water 15 is added to the container 11 with the common case 31 as shown in Fig. 9, the drinking water 15 is reacted with the hydrogen generating solid matter, and the calcium sulfate is dissolved and the active hydrogen is generated in a neighboring region.
In Embodiment 2, the active hydrogen is generated in the vicinity of a hydrated calcium ion and a sulfate ion dissociated from the calcium sulfate. Thus, the generated active hydrogen is efficiently bonded to the hydrated ion. Further, magnesium hydroxide formed on the magnesium particle surface is efficiently removed by the sulfate ion generated in the vicinity of the magnesium particles. A high-concentration, active hydrogen-dissolved water can be generated continuously in this manner.

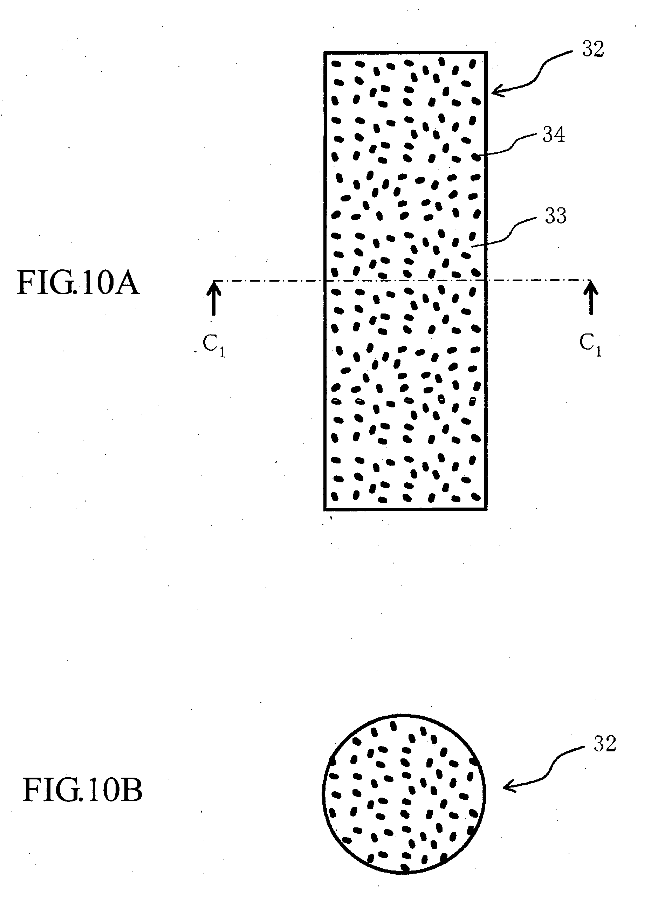

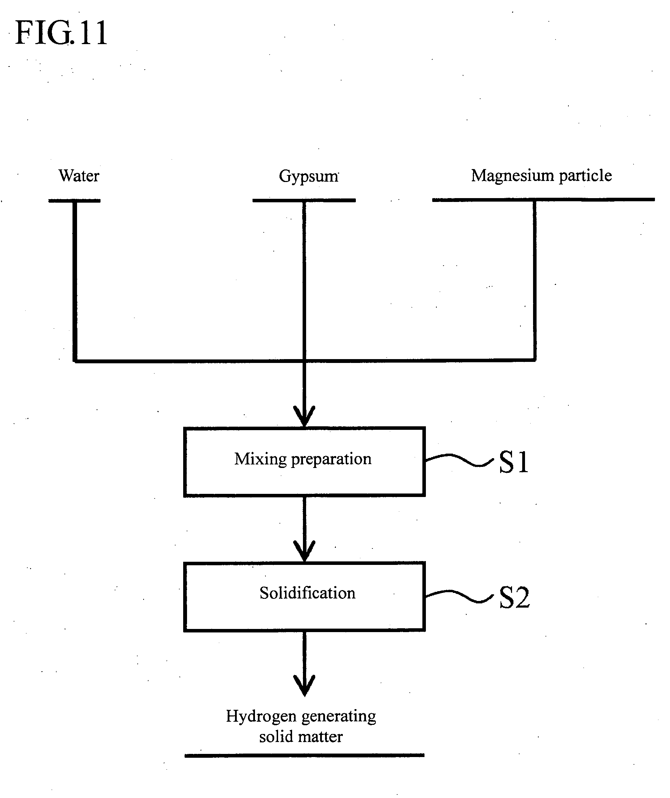

Next the hydrogen generating solid matter 32 will be described with reference to Figs. 10 and 11. Fig. 10A is a side view showing the hydrogen generating solid matter 32, and Fig. 10B is a sectional view along the C₁-C₁ arrow line in Fig. 10A. For example, the cylindrical hydrogen generating solid matter 32 may be composed of 70 g of a solid gypsum 33 and approximately 1 g of a powdery magnesium particles 34. The magnesium particles 34 preferably have a particle diameter of 0.05 to 0.15 mm, and is mixed with the gypsum such that the particles are substantially uniformly dispersed in the gypsum.
In the case of using such a structure, the hydrogen generating solid matter 32 can be uniformly eluted into the drinking water 15 in the generation of the active hydrogen-dissolved water. Thus, the dissolution of the gypsum and the reaction consumption of the magnesium particles are caused at substantially the same rates in a balanced manner. There is no case where only the gypsum in the hydrogen generating solid matter 32 is dissolved and the magnesium particles remain therein, and there is no case where the gypsum is hardly dissolved and the magnesium particles are not reacted with the drinking water 15.

The method for producing the hydrogen generating solid matter 32 will be described with reference to Fig. 11. Fig. 11 is a flow chart for illustrating the production method.
As shown in Fig. 11, water, powdery gypsum, and powdery magnesium particles are weighed respectively, and a thick mixture is prepared in Step S1 of mixing preparation. It is preferred that the water be boiled once and then made alkaline with sodium hydroxide, calcium hydroxide, or potassium hydroxide, etc. before the mixing. The pH value of the water is preferably controlled at approximately 10 to 13. The gypsum may be a hemihydrate gypsum, etc. As described above, it is preferred that magnesium particles having a diameter of 0.05 to 0.15 mm are classified and mixed. For example, 70 g of the gypsum is mixed with 0.9 to 1.2 g of the magnesium powder, and 2 to 5% by mass of the water is added thereto to prepare the thick mixture, the amount of water being determined depending on the viscosity. The first temperature of the preparation temperature is controlled at 10°C to 18°C. The first temperature is particularly preferably about 15°C.

Then, in Step S2 of solidification, the thick mixture is solidified at a second temperature, which may be room temperature (20°C to 25°C). It is preferred that, when the thick mixture is solidified into a clayey material, a pressure of 5 to 20 g/cm² is applied to the material and thus generated hydrogen gas is removed. The second temperature for the solidification is higher than the first temperature for the preparation. The hydrogen generating solid matter 32 is produced in this manner. The hydrogen generating solid matter 32 may have various shapes other than the cylindrical shape, and may have a rectangular or circular cylinder shape.

The hydrogen generating solid matter 32 produced in the manner of Fig. 11 is a dense mixture of the gypsum and magnesium particles. In the production of the mixture, it is the most important to make the first temperature lower than the second temperature, thereby preventing hydrogen generation in the production.

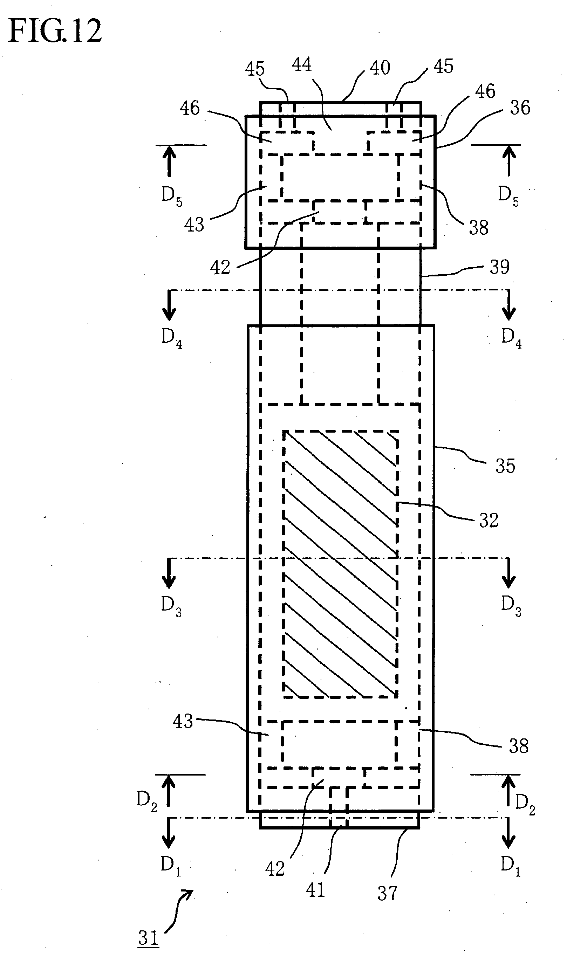

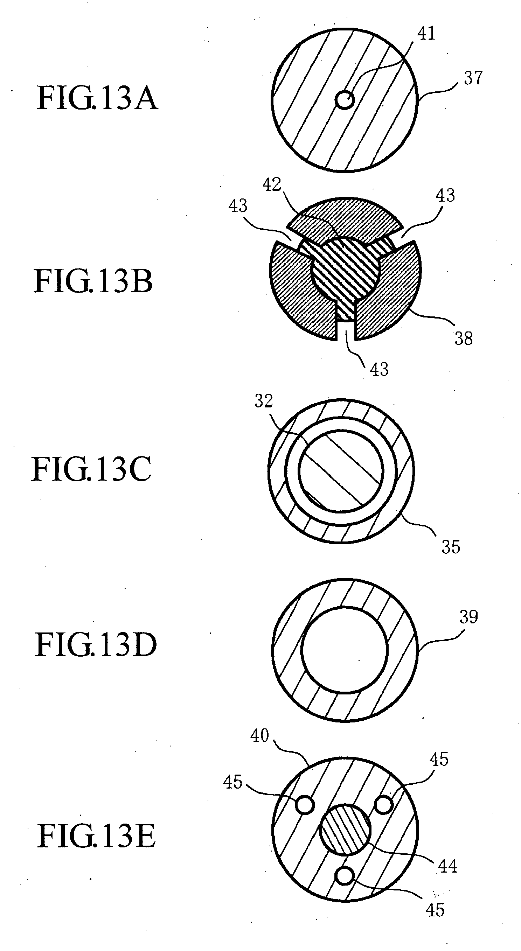

The common case 31 according to the preferred embodiment, in which the hydrogen generating solid matter 32 is placed, will be described with reference to Figs. 12 and 13, it being represented by oblique lines in the drawings. Fig. 12 is a side view of the common case 31. Fig. 13A is a transverse sectional view along the D₁-D₁ arrow line in Fig. 12, Fig. 13B is a view along the D₂-D₂ arrow line, and Fig. 13C is a transverse sectional view along the D₃-D₃ arrow line. Further, Fig. 13D is a transverse sectional view along the D₄-D₄ arrow line in Fig. 12, and Fig. 13E is a view along the D₅-D₅ arrow line.

As shown in Fig. 12, the common case 31 has a first cylindrical tube 35, in which the hydrogen generating solid matter 32 is contained, and a second cylindrical tube 36. A first member 37 and a second member 38 are attached to the bottom of the first cylindrical tube 35. An end of a third member 39 is removably attached to the top of the first cylindrical tube 35 in the same manner. Further, the other end of the third member 39 is attached to the bottom of the second cylindrical tube 36, and a second member 38 and a fourth member 40 are attached to the top of the second cylindrical tube 36. Each of the cylindrical tubes and members may be composed of a visible light shielding material such as vinyl chloride.

As shown in Figs. 12 and 13A, a hole 41 is formed as a water flow channel in the first member 37. As shown in Figs. 12 and 13B, a depression 42 is formed at the center of the second member 38, and flow channels 43 are formed at 3 positions in the periphery. In this structure, the drinking water 15 passes through the hole 41, the depression 42, and the flow channels 43, and reaches the hydrogen generating solid matter 32 shown in Figs. 12 and 13C.

As shown in Figs. 12 and 13D, the third member 39 has a cylindrical shape. Further, a protrusion 44 is formed at the center of the fourth member 40, and gas venting holes 45 are formed in 3 positions around the protrusion 44, as shown in Figs. 12 and 13E.

Then, method of using the common case 31 of the generator 10a according to Embodiment 2 for generating the active hydrogen-dissolved water will be described with reference to Figs. 9, 12, and 13.

As shown in Fig. 9, the common case 31 is stood on the first member 37 in the container 11 of the PET bottle, and the container 11 is filled with a tap water. It is preferred that chlorine in the tap water is removed by a commercially-available simple water purifier, etc. By storing the PET bottle in a room or a refrigerator for about half a day while protecting from direct sunlight, a sufficiently good-tasting active hydrogen-dissolved water can be generated. Then, this active hydrogen-dissolved water is used for drinking. When the active hydrogen-dissolved water in the PET bottle is used up, a tap water may be added to the PET bottle to continue the use of the active hydrogen-dissolved water.

In the generation of the active hydrogen-dissolved water, the drinking water 15 is introduced into the common case 31 and reacted with the hydrogen generating solid matter 32 in the container 11. The gypsum 33 is gradually dissolved from the solid matter surface, and the magnesium particles 34 are uniformly reacted and consumed. The active hydrogen is generated according to Chemical Formula 1, and part of the active hydrogen is bonded to a hydrated calcium ion dissociated in the vicinity, and dissolved in the drinking water 15. However, most of the active hydrogen is converted to a hydrogen molecule according to Chemical Formula 1, and the resultant hydrogen gas is moved through the third member 39 upward. The hydrogen gas passes through the depression 42 and the gas flow channels 43 in the second member 38, and reaches a gas reservoir 46. The gas reservoir 46 is a space between the second member 38 and the fourth member 40.
As the reaction of Chemical Formula 1 proceeds, the more hydrogen gas is accumulated in the gas reservoir 46 and the hydrogen gas pressure is increased, so that the drinking water containing a high level of the active hydrogen in the common case 31 is pushed downward and eluted off the common case 31 through the hole 41. When the hydrogen gas pressure is further increased to exceed the critical value, the hydrogen gas is vented at once from the hole 45. Then, the drinking water 15 in the container 11 back-flows and is introduced through the hole 41 into the common case 31, and the drinking water containing a high level of the active hydrogen is generated again according to the above mentioned mechanism. By repeating the processes, the active hydrogen-dissolved water generated in the common case 31 is accumulated in the container 11.

In Embodiment 2, even if the common case 31 and the drinking water 15 are put in the container 11 and stored for 2 days, the drinking water 15 has a water hardness of 80 or less. Further, the drinking water has a pH value of about 7.5. The active hydrogen-dissolved water generated using the generator 10a in this manner is sufficiently good-tasting water.

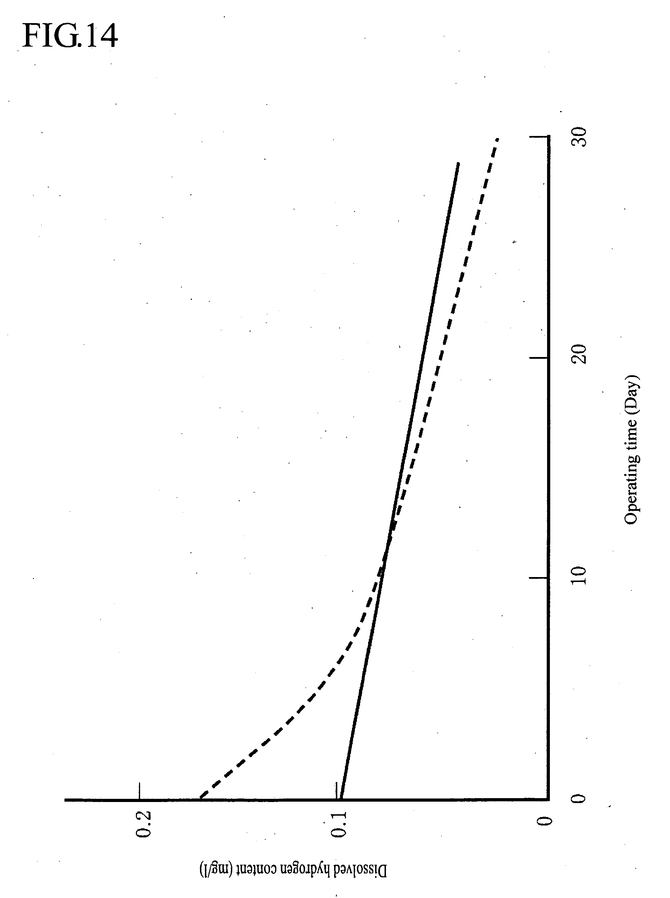

An effect obtained in Embodiment 2 will be described with reference to Fig. 14. In Fig. 14, Embodiment 2 is compared with a conventional method of soaking only a magnesium metal piece in a commercially-available tap water. Fig. 14 is a graph showing temporal changes in the dissolved hydrogen contents (the total contents of hydrogen molecule and active hydrogen) of active hydrogen-dissolved waters, similarly to Fig. 8.

In Fig. 14, the solid line represents an example of the results of soaking and dissolving the hydrogen generating solid matter 32 of the gypsum magnesium mixture. In this case, the amount of dissolved calcium sulfate corresponds to 7 × 10⁻⁴ mol/l in Fig. 7, and the water hardness is about 70 after dissolution. The dotted line represents the results of the conventional method of soaking only the magnesium metal particle in the commercially-available tap water.
In the conventional method, the hydrogen generation is remarkably reduced in 10 days of the operating time as described for Fig. 8. In contrast, in the case of dissolving the hydrogen generating solid matter 32, the dissolved hydrogen content is reduced at a small constant rate, resulting in a higher retention of the reaction represented by Chemical Formula 1. In this case, the dissolved hydrogen content is reduced because the surface of the hydrogen generating solid matter 32 is eluted over the operating time to decrease its surface area. As a result of observing the surface of the in-use hydrogen generating solid matter 32 by a microscope, substantially no magnesium hydroxide is formed according to Chemical Formula 2 on the surface of the magnesium particle 34.

Thus, the retention of the active hydrogen generation is improved in Embodiment 2 more than in Embodiment 1. Also in this case, the process of soaking the magnesium particle 16 in a vinegar after using the first case 12 for a predetermined time, thereby dissolving and removing the magnesium hydroxide, is not required at all, though the process is necessary in the conventional method. Further, in this case, the active hydrogen content of the active hydrogen-dissolved water is higher than that of the conventional method, similarly to the description of Fig. 7.
The common case 31 used in Embodiment 2 is obtained by assembling a plurality of members. Thus, when the hydrogen generating solid matter 32 is used up, another hydrogen generating solid matter 32 may be put in the common case 31 and used.

### (Embodiment 3)

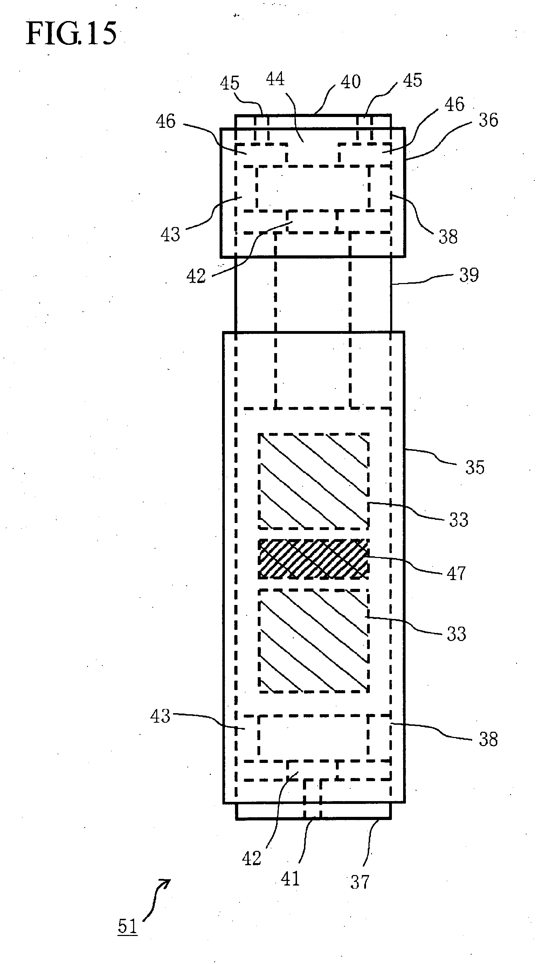

Embodiment 3 will be described with reference to Fig. 15. In this embodiment, the magnesium metal and gypsum for the active hydrogen generation are not mixed and are separately put in a common case 51.

The common case 51 is composed of a thin cylindrical plastic material, etc., and has such a structure that a magnesium metal 47 and gypsums 33 are alternately placed as shown in Fig. 15.

In Embodiment 3, the common case 51 is put in a container 11 filled with a drinking water 15 to generate the active hydrogen-dissolved water as shown in Fig. 9. The drinking water 15 is introduced from a hole 41, and reacted with the magnesium metal 47 in an enclosing chamber 35 according to Chemical Formula 1 to generate the active hydrogen. The dihydrate gypsum 33 in the enclosing chamber 35 is dissolved in the drinking water 15 introduced from the hole 41, to generate a dissociated, hydrated calcium ion. The hydrated calcium ion captures the active hydrogen.

In this case, a region for generating the active hydrogen is extremely close to a region for generating the hydrated calcium ion. Thus, the generated active hydrogen is efficiently captured by the hydrated calcium ion, and most of the active hydrogen is bonded to the active hydrogen adsorption ion. As a result, the effects of the generated active hydrogen-dissolved water are maintained in a longer period. In this case, it is necessary to control the amounts of the magnesium metal 47 and the gypsum 33 such that they are usable in substantially the same periods.

In Embodiment 3, the gypsum 33 may be dihydrate gypsum or anhydrous gypsum.

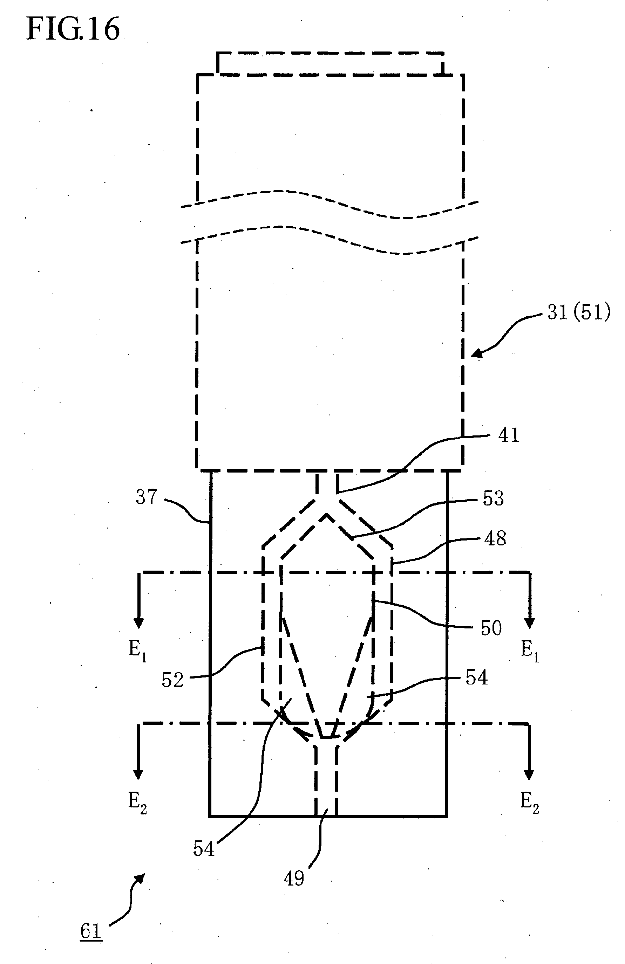

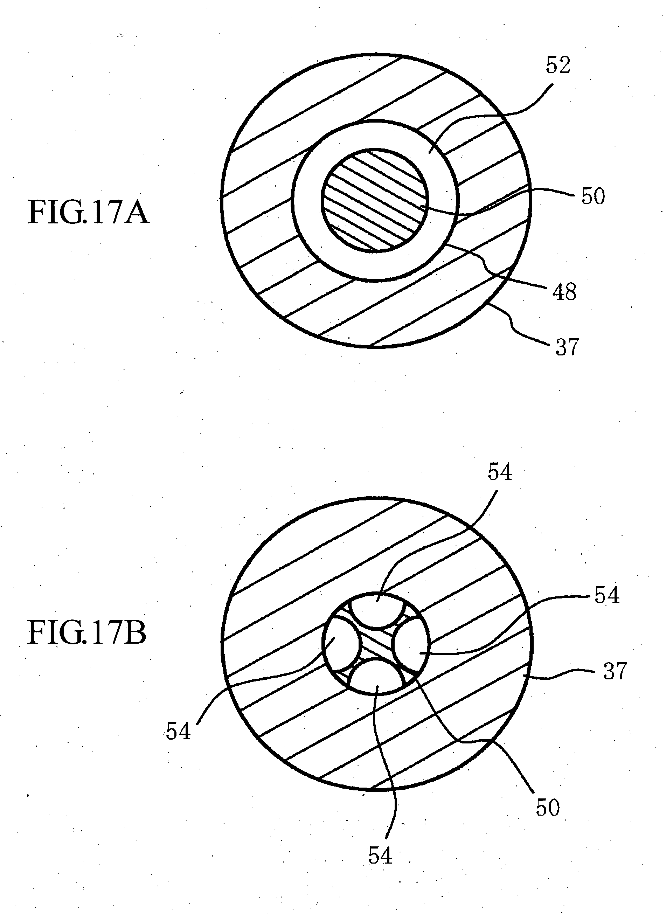

A modified example of the common cases 31 and 51 used in the above embodiments will be described with reference to Figs. 16 and 17. Fig. 16 is a side view showing a common case according to the modified example. Figs. 17A and 17B are transverse sectional views along the E₁-E₁ arrow line and the E₂-E₂ arrow line in Fig. 16, respectively.

As shown in Fig. 16, in a common case 61, a first member 37 of the common case 31, 51 described in Embodiments 2 and 3 is expanded, and a valve chamber 48 connected to a hole 41 is formed in the first member 37. A hole 49 connected to the valve chamber 48 is formed on the edge of the first member 37, and acts as a flow channel for the drinking water 15 in the generator 10a. Further, a needle valve 50 movable up and down, composed of a metal, etc., is placed in the valve chamber 48.

For example, as shown in Figs. 16 and 17A, the valve chamber 48 has a cylindrical shape, and has conic surfaces with reducing diameters at the upper and lower ends, which are connected to the holes 41 and 49 respectively. The valve 50 has a cylindrical body corresponding to the shape of the valve chamber 48, and a toric gap 52 is formed between the valve 50 and the valve chamber 48. The valve 50 has an upper end of a conically shaped protrusion 53, and has a convex lower end, which is in contact with the tapered region in the valve chamber 48. Further, as shown in Figs. 16 and 17B, a part of the valve 50 is bored such that grooves 54 extends from the cylindrical body to the lower end of the valve 50. The number of the groove 54 may be 1 or more, though 4 grooves 54 are formed in this example.

The use of the common case 61 and the generation of the active hydrogen-dissolved water using the common case 61 are substantially equal to those in Embodiment 2. When the common case 61 is stood on the first member 37 in the container 11 of the PET bottle in the same manner as the common case 31, the valve 50 is moved to and comes into contact with the tapered region in the lower end of the valve chamber 48 because the valve 50 is made of a metal to be heavy. The gap 52 and the hole 49 are connected via the grooves 54 formed on the periphery of the valve 50, so that the drinking water 15 in the container 11 is introduced through the hole 49, the grooves 54, the gap 52, and the hole 41 to the common case 31 or 51. The active hydrogen-dissolved water generated in the common case 31 or 51 is introduced through the hole 41, the gap 52, the grooves 54, and the hole 49 to the container 11 in the reverse direction.

The valve 50 acts due to its own weight to stably maintain the first member 37 of the common case 61 on the downside in the container 11. Therefore, the common case 31 or 51 can generate the active hydrogen-dissolved water extremely stably.

When the common case 61 is put in the container 11 such that the first member 37 is on the upper side, the valve 50 is moved to the upper end of the valve chamber 48 due to its own weight, the protrusion 53 comes into contact with the tapered region in the upper end of the valve chamber 48. Thus, the connection between the hole 41 and the valve chamber 48 is closed. The generation of the active hydrogen-dissolved water by the common case 31 or 51 is stopped or inhibited in this manner, and the safeness of the generation is improved. The material of the valve 50 is not limited to a metal, and may be vinyl chloride, a plastic, etc.

The embodiments of the invention are described in detail above with reference to the drawings. The configuration of the invention are not limited to the embodiments, and modifications may be made thereto without departing from the scope of the invention.

For example, in Embodiment 1, the gypsum may be provided without using the second case 13 such that the first case 12 is put in the container 11, the container 11 is filled with a certain amount of the tap water, and a certain amount of the gypsum such as hemihydrate gypsum, dihydrate gypsum, or anhydrous gypsum is added to and dissolved in the tap water. For example, 200 mg of the calcium sulfate is dissolved in 2 L of the tap water. The active hydrogen-dissolved water may be prepared as a drinking water by mixing a certain amount of the calcium sulfate and by storing for a predetermined period (e.g. half a day) in this manner. In this case, the active hydrogen-dissolved water in the container 11 is used until no water remains. When the water is consumed, the mixing process is carried out again to prepare the active hydrogen-dissolved water. Magnesium sulfate may be used instead of calcium sulfate.
A tablet of the calcium sulfate or magnesium sulfate is preferably used in the method of mixing and dissolving a certain amount of the calcium or magnesium sulfate, thereby generating the active hydrogen-dissolved water.

For example, in Embodiment 2, the active hydrogen-dissolved water for drinking may be obtained without using the common case 31 such that a tablet of the hydrogen generating solid matter is directly added by a certain amount to the drinking water and stored for a predetermined period (e.g. half a day). In this case, the gypsum, with which the magnesium metal powder is mixed, may be hemihydrate gypsum, dihydrate gypsum, or anhydrous gypsum.

In the above embodiments, a magnesium sulfate solid may be used instead of the gypsum, and a hydrated magnesium ion dissociated therefrom may be utilized. In this case, the second case 13 or the common case 31, 51 is filled with the magnesium sulfate solid or a solid mixture of the magnesium sulfate and magnesium metal powder. Alternatively, the case may be filled with a mixture of the calcium sulfate solid and the magnesium sulfate solid.

The gypsum supply member used in the above embodiments may have various shapes. Though the gypsum supply member has a thin cylindrical shape in the embodiments, the member is not limited thereto and may be a tabular case filled with the gypsum.

## Claims

1. A method for generating an active hydrogen-dissolved water, **characterized by** adding magnesium metal, and gypsum or magnesium sulfate to water, thereby generating a water containing an active hydrogen.

2. A method for generating an active hydrogen-dissolved water, **characterized by** adding a mixture of magnesium metal and gypsum to water, thereby generating a water containing an active hydrogen.

3. The method for generating an active hydrogen-dissolved water according to claim 1 or 2, **characterized in that** the gypsum is hemihydrate gypsum, dihydrate gypsum, or anhydrous gypsum.

4. An active hydrogen-dissolved water generator comprising a water container, a member filled with magnesium metal which is placed in the container, and a member filled with gypsum which is placed in the container.

5. An active hydrogen-dissolved water generator comprising a water container and a member filled with magnesium metal and gypsum which is placed in the container.

6. An active hydrogen-dissolved water generator comprising a water container and a member filled with a mixture of magnesium metal and gypsum which is placed in the container.

7. An active hydrogen-dissolved water generator comprising a water container, a member filled with magnesium metal which is placed in the container, and a member filled with magnesium sulfate which is placed in the container.

8. The active hydrogen-dissolved water generator according to claim 4, 5, or 6, **characterized in that** a light shielding layer is formed on a surface of the member to prevent the gypsum from being exposed to light.

9. A gypsum supply member for the method for generating an active hydrogen-dissolved water according to claim 1, 2, or 3, comprising a case into which water is introduced, and gypsum or a mixture of magnesium metal and gypsum put in the case.

10. The gypsum supply member according to claim 9, **characterized in that** a light shielding layer is formed on a surface of the case to prevent the gypsum from being exposed to light.

11. An active hydrogen generating material comprising a solid matter prepared by mixing a magnesium metal powder with gypsum, the material being added to water to generate an active hydrogen.

12. The active hydrogen generating material according to claim 11, **characterized in that** the magnesium metal powder has a particle diameter of 0.05 to 0.15 mm.

13. The active hydrogen generating material according to claim 11 or 12, **characterized in that** the gypsum is hemihydrate gypsum, dihydrate gypsum, or anhydrous gypsum.

14. A method for producing the active hydrogen generating material described in claims 11 to 13, **characterized by** comprising the steps of mixing gypsum, a magnesium metal powder, and water at a first temperature to form a thick mixture, and solidifying the thick mixture at a second temperature higher than the first temperature.

15. The method for producing the active hydrogen generating material according to claim 14, **characterized in that** the gypsum is in a powdery form.

16. The method for producing the active hydrogen generating material according to claim 14 or 15, **characterized in that** the first temperature is within the range of 10°C to 18°C, and the second temperature is room temperature.

17. The method for producing the active hydrogen generating material according to claim 14, 15, or 16, **characterized in that** the water has a pH value of 10 to 13.

18. The method for producing the active hydrogen generating material according to claim 17, **characterized in that** sodium hydroxide, calcium hydroxide, or potassium hydroxide is dissolved in the water.

19. The method for producing the active hydrogen generating material according to claim 17 or 18, **characterized in that** the water is boiled once before the mixing step.

20. A method for producing the active hydrogen generating material, **characterized in that** the step of solidifying the thick mixture at the second temperature comprises pressure solidification under a pressure of 5 to 20 g/cm².
